# FASCICULE DE BREVET EUROPEEN

(11) **EP 0 871 387 B1**
(45) Date de publication et mention de la délivrance du brevet: **05.07.2000**
(21) Numéro de dépôt: 96941152.9
(22) Date de dépôt: 23.12.1996
(51) Int. Cl.: A47G 21/08, B26B 25/00

(54) **DISPOSITIF DE TABLE POUR HANDICAPES PHYSIQUES**
ESSGERÄT FÜR BEHINDERTE
TABLE ATTACHMENT FOR THE PHYSICALLY HANDICAPED

(30) Priorité: 29.12.1995 CH 371295; 08.07.1996 CH 168996
(43) Date de publication de la demande: 21.10.1998
(73) Titulaire: Stucky, Eric, CH-2300 La Chaux-de-Fonds (CH)
(72) Inventeur: Stucky, Eric, CH-2300 La Chaux-de-Fonds (CH)
(86) Numéro de dépôt international: IB9601460
(87) Numéro de publication internationale: WO9724050

(56) Documents cités:
- DE-U- 8 527 734
- DE-U- 8 903 346

## Description

L'invention a pour objet un dispositif de table pour handicapés physiques, et un appareil pour sa recharge.

Le manque de force dans les bras ou les mains, l'impossibilité de s'aider avec les objets et l'imprécision du mouvement de certains handicapés, voire de personnes âgées, augmente leur dépendance, ceci également lors des repas. En effet, la préparation des aliments dans l'assiette nécessite très souvent l'intervention d'une tierce personne; la coupe d'une tranche de viande, par exemple, est impossible à certains handicapés.

Ce problème a déjà préoccupé certains inventeurs, par exemple dans le document DE-U-8903346 il est décrit un dispositif pour handicapés physiques à motorisation et source d'énergie électrique intégrée, destiné à couper une pièce de nourriture comportant un boîtier oblong pouvant être tenu d'une main comprenant un organe de coupe, présentant une partie tranchante, en prise avec des moyens associés à un moteur électrique permettant de mettre en rotation l'organe de coupe, ainsi que des moyens pour immobiliser la pièce à découper lors de la rotation dudit organe. Ce dispositif est compliqué et son utilisation est délicate. En effet, pour couper un morceau de viande l'utilisateur doit déplacer le dispositif, c'est-à-dire le soulever en décrochant la pièce à découper et le replanter dans l'alignement de la coupe précédente.

L'invention a pour but de fournir un dispositif de table pour handicapés physiques du type décrit ci-devant tendant à réduire leur dépendance, notamment de leur permettre de découper un aliment, plus particulièrement un morceau de viande, et de le porter à leur bouche.

Le dispositif de table pour handicapés défini à la revendication 1 atteint ce but.

Pour pallier l'absence de force de l'utilisateur du dispositif selon l'invention ce dernier emploie une énergie auxiliaire fournie par un mini-moteur avec une source d'alimentation intégrée. De plus, ledit dispositif devra comprendre un moyen de mise en marche de sécurité ne permettant que son enclenchement au moment opportun.

Le dispositif selon l'invention réalise un ensemble de fonctions qu'une personne en bonne santé exécute instinctivement. Ainsi, la coupe d'un morceau de viande, par exemple, nécessite la suite des opérations suivantes :
- immobiliser le morceau avec une fourchette;
- actionner le couteau avec discernement. En effet, pour une séparation totale des deux parties,il est impératif qu'une pointe de lame au moins parcourt la longueur totale de la coupe en étant en contact avec la surface de l'assiette. Ce n'est qu'à cette condition que les deux parties se séparent. Une personne valide fera glisser la lame à l'endroit exact; cela ne va pas de soi pour un handicapé physique;
- une fois coupé de part en part, la fourchette devra être dégagée du morceau. Le plus souvent le couteau servira d'extracteur;
- la partie coupée sera reprise avec la fourchette.

Le but visé par l'invention est de fournir un dispositif permettant de faire toutes les opérations définies d'une seule main, c'est-à-dire de dégager une bouchée par coupe et ceci avec une seule main.

L'invention sera mieux comprise et son principe apparaîtra plus clairement à la lecture de la description de plusieurs formes d'exécution données à titre d'exemples en regard des dessins sur lesquels :
la figure 1 représente une forme d'exécution du dispositif selon l'invention en coupe longitudinale et quatre coupes transversales,
la figure 2 représente quatre formes d'exécution présentant différentes positions de l'organe de coupe,
la figure 3 représente une autre variante du dispositif selon l'invention,
la figure 4 représente une forme d'exécution du contact de sécurité pour le dispositif selon l'invention,
la figure 5 représente une forme d'exécution de l'appareil de recharge du dispositif de l'invention.
Les caractéristiques semblables seront désignées par les mêmes signes de référence dans toutes les figures

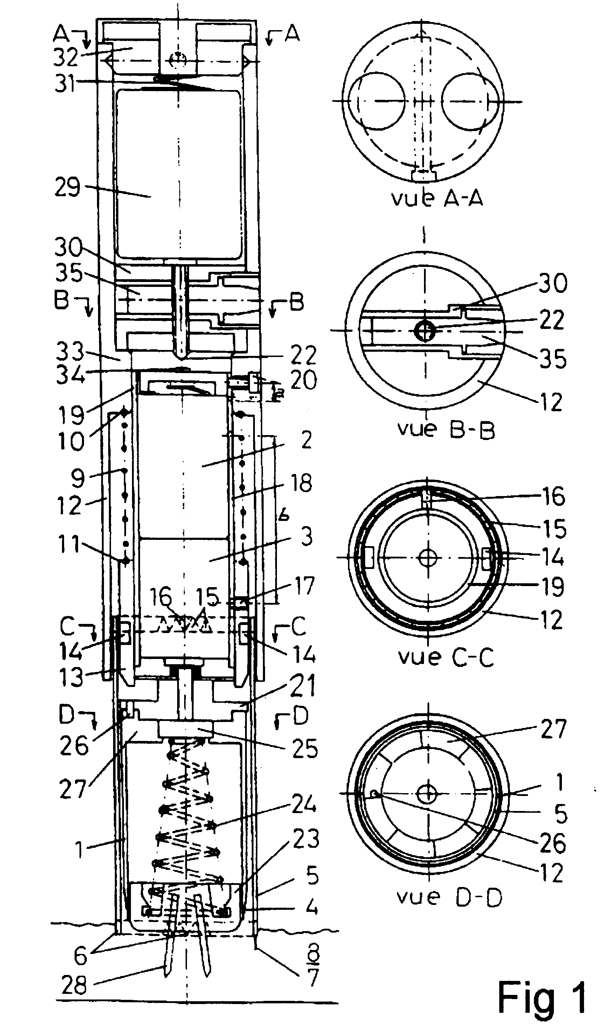

Comme on le voit sur la figure 1, un dispositif selon l'invention comprend un boîtier oblong 12 comportant à une de ses extrémités un organe de coupe 1. L'organe de coupe 1 est constitué d'un cylindre circulaire ayant l'extrémité distale 4 sous forme de tranchant qui peut comporter des dents. L'extrémité proximale est constituée par un fond 27 dont la partie centrale est constituée par un aimant 25 destiné à maintenir l'organe de coupe 1 contre le plateau 21. Le plateau 21 est directement lié en rotation au groupe moto-réducteur comprenant un moteur 2 et un réducteur 3. La rotation de l'organe de coupe 1 est assurée par une goupille 26 liée au plateau 21 venant s'encliqueter dans une cannelure du fond 27. La vitesse et le couple sont adaptés de manière à obtenir une pénétration optimale de l'organe de coupe dans la pièce de nourriture à couper.

Les moyens pour immobiliser la pièce 8 à découper, qui constituent ce que nous nommerons l'effet fourchette, comprennent un cylindre 5 circulaire concentrique extérieurement à l'organe de coupe 1. Le cylindre 5 comporte à sa partie distale des dents 6 et dans la forme d'exécution décrite des pics 7 qui viennent se planter dans la pièce à découper 8. Le cylindre 5 est fixe dans le sens de la rotation et mobile dans le sens axial relativement au boîtier 12. La fixation en rotation du cylindre 5 est réalisée par une denture 15 disposée à l'extrémité proximale dudit cylindre 5 qui est maintenu contre une goupille 16 solidaire d'un porte-cylindre 13 par des aimants 14, deux dans la forme d'exécution décrite.

Le porte-cylindre 13 est maintenu fixe en rotation relativement au boîtier cylindrique 19 du groupe moto-réducteur 2 et 3 par une clavette 17 se déplaçant dans une fente axiale 18 dudit boîtier 19. Ces dispositions entraînent que le porte-cylindre 13 et donc le cylindre 5 sont solidaires en rotation avec le boîtier 19 du groupe moto-réducteur, mais mobiles axialement par rapport audit boîtier 19.

Le porte-cylindre 13 est maintenu par un ressort 9 s'appuyant dans des logements 10 et 11 prévus sur le boîtier oblong 12 et sur le porte-cylindre 13 dans une position basse lorsque le dispositif selon l'invention est tenu verticalement ou en position sortie relativement au boîtier 12. Le boîtier 19 est maintenu fixe en rotation avec le boîtier 12 par une clavette 20 qui se déplace dans une fente axiale dudit boîtier 19 du groupe moto-réducteur 2 et 3.

Le déplacement axial maximal du porte-cylindre 13 par rapport au boîtier 19 a une longueur "b" représentée sur la figure 1, ce qui correspond à l'épaisseur maximale que le dispositif selon l'invention peut découper.

Le déplacement axial maximal du boîtier 19 par rapport au boîtier oblong 12 a une longueur "a" représentée sur la figure 1, ce qui correspond au déplacement du contact 34 du moteur venant toucher le contact 22 de l'alimentation.

Lorsque l'opérateur ayant le boîtier oblong 12 en main exercera une légère pression verticale vers le bas, l'ensemble moto-réducteur 2 et 3 solidaire du boîtier cylindrique 19 va se déplacer vers le haut assurant la fermeture du contact 22 - 34 qui alimente le moteur.

Le maintien d'une pression vers le bas comprime le ressort 9 ; ce dernier, s'appuyant sur la porte-cylindre 13 puis sur le cylindre 5, permettra à celui-ci de reculer progressivement vers le haut à la vitesse de pénétration de l'organe de coupe dans la nourriture, maintenant un contact permanent entre ses dents 6, neutralisant le couple dudit organe 1 exercé sur la nourriture à couper 8.

Remarquons que le moto-réducteur actionne l'organe 1, alors que la nourriture 8 est déjà immobilisée par les dents 6 du cylindre extérieur 5. Lorsque la coupe est effectuée, l'opérateur, en retirant le boîtier 12 vers le haut, dégage l'organe 1 et le cylindre 5 provoquant l'arrêt du moteur.

On peut remarquer que l'organe de coupe 1 est toujours protégé soit par le cylindre 5 au repos, soit par la nourriture lorsque l'organe de coupe est en fonction.

Le cylindre 5 peut être séparé du porte-cylindre 13 pour le nettoyage. Cette séparation s'opère en retirant le cylindre retenu par un dispositif à encliquetage mécanique ou magnétique. De même, l'organe de coupe 1 doit pouvoir être extrait et réintroduit sans causer de difficulté à l'opérateur. Le dispositif d'encliquetage et d'entraînement de l'organe de coupe sera décrit ci-après.

Le système décrit ci-devant ne serait que partiellement opérationnel s'il n'était pas complété par un dispositif d'extraction de la nourriture coupée, laquelle, sans ce dernier, resterait prisonnière de l'organe de coupe.

L'organe de coupe 1 présente à son intérieur un piston 23 en matériau conforme aux exigences en matière d'alimentation ; ce dernier est tenu par un ressort 24 approximativement au niveau de la dentelure 4. Ledit piston est creux de manière à pouvoir contenir le ressort en position de compression ; ce dernier est fixé au piston 23.

La boucle supérieure du ressort 24 est tenue par une pastille aimantée 25 solidaire de la partie supérieure de l'organe de coupe 1. De cette manière, l'enlèvement du couteau est réalisable sans outils et avec peu de force. En particulier, la remise en place de l'organe de coupe ne nécessite aucune précision puisque le couteau est attiré par l'aimant et se centre de lui-même ; il suffit de l'introduire à l'intérieur du cylindre 5 et de le pousser.

En équipant le piston 23 de la figure 1 d'au moins une dent de fourchette 28, l'opérateur n'aura pas à reprendre un autre couvert pour porter à la bouche le morceau de nourriture coupé. En effet, en plaçant le dispositif de table sur la partie à découper, la ou les dents pénétreront dans la nourriture au plus tard lorsque le piston sera en fin de course haute. Ceci implique une pénétration de l'organe de coupe dans la nourriture d'une certaine profondeur bien entendu. En retirant ledit dispositif, la nourriture poussée vers le bas par le piston sera à disposition de l'opérateur qui pourra la porter à la bouche sans changer de couvert et sans danger.

Pour adapter l'outil au plus grand nombre de cas possibles, la ou les dents 28 de la fourchette centrale peuvent être fixées mécaniquement ou magnétiquement sur la partie externe du piston 23 de l'extracteur utilisant le même principe que celui de la fixation de l'organe de coupe 1.

La ou les batteries d'alimentation 29 et le bloc de recharge en matière isolante 30 de la figure 1 sont contenus dans la partie supérieure du boîtier 12, pressés vers le bas par un ressort 31 s'appuyant sur la fermeture 32, l'ensemble étant retenu vers le bas par une portée intérieure 33 du boîtier 12 qui détermine sa position.

Lors d'une pression du boîtier vers le bas, l'ensemble organe de coupe et moto-réducteur monte, le contact 34 du moteur touche la borne d'alimentation 22 de la batterie, mettant en marche le moteur, son pôle positif étant à la masse.

La recharge de l'accumulateur se fait par l'intermédiaire de la borne 35 qui est reliée électriquement au contact 22 lui-même relié au pôle négatif de la batterie, son pôle positif étant à la masse. Cette recharge sera décrite ci-après.

Quel que soit le type de couteau, manuel linéaire, linéaire motorisé ou rotatif motorisé, la coupe de part en part d'un aliment nécessite impérativement qu'au moins un point de la lame soit en contact avec l'assiette sur toute la longueur de la coupe, dans le présent cas au moins 360 degrés.

En effet, la pression de la main sur un point d'une surface très petite se traduit par une pression spécifique considérable indispensable pour la coupe de part en part de certains aliments.

Dans le cas du dispositif de table pour handicapés ou couteau rotatif, cette condition ne sera remplie que si la lame est perpendiculaire à la surface de l'assiette. Pour un handicapé dont la mobilité est limitée, il faut accepter une non perpendicularité de la lame. Une correction doit donc être assurée automatiquement.

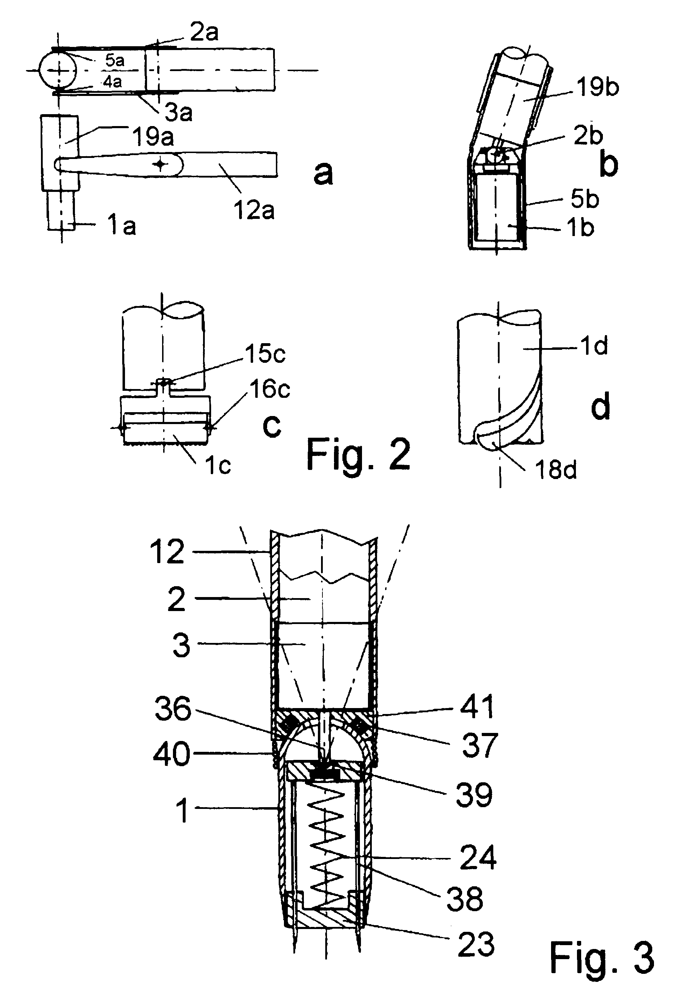

Plusieurs manières peuvent être envisagées. La figure 2 montre quatre formes d'exécution a, b, c et d pouvant conduire à ce résultat.

La figure 2a représente une exécution dans laquelle l'ensemble moto-réducteur 19a et l'organe de coupe ou lame 1a sont montés sur deux articulations 4a, 5a lesquelles sont tenues par deux bras 2a, 3a eux-mêmes articulés de manière indépendante par rapport au manche 12a. Ainsi, le système de coupe peut osciller selon deux axes et s'adapter au plan de l'assiette de manière à trancher de part en part. Dans cette forme d'exécution, la batterie peut être logée dans ledit manche, chaque bras articulé tenant lieu de conducteur d'alimentation.

La figure 2b représente une exécution dans laquelle la lame 1b est entraînée par une rotule 2b solidaire du réducteur 19b. Cette dernière comporte une goupille d'entraînement s'engageant dans un cylindre fendu solidaire de la lame ; la rotule, en matériau magnétique, est maintenue au fond dudit cylindre par une pastille aimantée. Ainsi l'axe du moto-réducteur et celui de la lame peuvent ne pas être alignés et cette dernière s'adapter au plan de l'assiette.

Dans cette exécution, le cylindre concentrique denté 5b immobilisant la nourriture devra pouvoir lui aussi s'adapter à l'angle formé par les deux axes, tout en exerçant la pression requise sur la nourriture à couper et rentrant progressivement à l'intérieur de la poignée.

Une forme d'exécution consiste à diviser le cylindre 5b en plusieurs éléments concentriques, s'emboîtant les uns dans les autres et articulés entre eux selon deux axes. Ils pourront s'adapter à l'inclinaison de l'organe de coupe ou de la lame 1b, condition nécessaire pour que ce dernier puisse s'adapter au plan de l'assiette.

La figure 2c représente une autre forme d'exécution dans laquelle l'organe de coupe comporte à son extrémité inférieure deux articulations 15c et 16c décalées de 90° tel un cardan ; il peut ainsi s'adapter à la planéité de l'assiette puisque le cylindre immobilisant la nourriture se sera retiré, dégageant l'organe de coupe qui peut ainsi s'orienter librement.

Dans cette exécution, la non perpendicularité de l'axe du manche par rapport à l'assiette peut être relativement importante.

La figure 2d représente une autre forme d'exécution dans laquelle l'organe de coupe 1d ou lame est taillé de manière à obtenir au moins un point de coupe 18d saillant et rétractable toujours en contact avec l'assiette en dépit de sa non perpendicularité; le dégagement hélicoïdal ou non de cet élément de lame lui conférant une certaine élasticité. Il donne la garantie qu'au moins un point parcourant les 360° entrera en contact avec l'assiette.

Une autre forme d'exécution visible sur la figure 3 visant à supprimer la couronne d'immobilisation des aliments consiste à utiliser une fourchette interne à l'organe de coupe.

Dans cette optique, il est indispensable d'obtenir à l'intérieur de l'organe de coupe 1 un point fixe 36 non existant dans l'exécution décrite ci-devant. Ce point fixe est obtenu par l'axe du réducteur 3, le boîtier de ce dernier tournant par rapport au moteur 2 solidaire du boîtier manche 12.

Le réducteur entraîne un fond hémisphérique concave 41 entraînant la partie supérieure convexe de même rayon que l'organe de coupe 1. Les deux calottes sont maintenues en contact par au moins un aimant 37, l'entraînement étant assuré par une goupille pouvant se déplacer dans un logement du fond de manière à permettre un certain angle entre les axes du boîtier manche 12 et de l'organe de coupe 1.

Le piston 23, poussé vers le bas par un ressort 24, est traversé par au moins une tige 38 solidaire du point fixe 36 par l'intermédiaire d'une articulation 39 située au centre du rayon de la calotte hémisphérique. Un moyen élastique ou à ressort 40, solidaire du boîtier 12, positionne l'organe de coupe 1 verticalement après usage.

Dans la forme d'exécution où une fourchette est incorporée à la lame de manière à porter la nourriture à la bouche d'un seul mouvement, le danger existe d'enclencher l'appareil dans cette position à la suite d'un mouvement malencontreux.

En fait le contact de sécurité, qui doit éviter tout accident, ne doit être fermé que lorsque le boîtier de l'appareil est approximativement vertical, seulement si la lame est dirigée vers le bas, alors qu'une pression verticale de haut en bas est exercée sur lui.

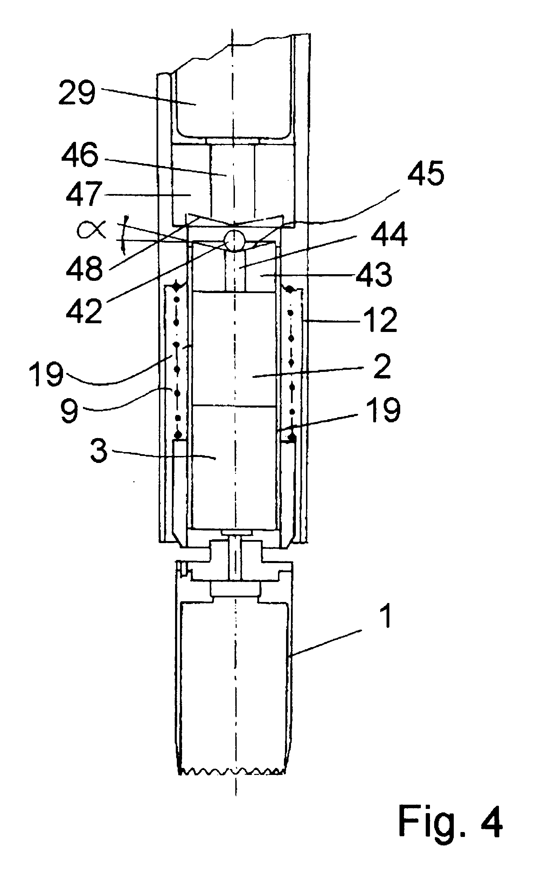

La figure 4 montre une forme d'exécution du dispositif permettant de remplir les conditions évoquées ci-dessus. Elle représente une coupe longitudinale de la partie du dispositif de table pour handicapés ou couteau rotatif intégrant le dispositif d'enclenchement.

Lorsque l'on pèse sur la lame 1, le tube 19, dans lequel se trouvent le moteur 2 et le réducteur 3, se déplace vers le haut par rapport au boîtier 12. Dans la partie supérieure du tube 19 est fixée une collerette en matériau isolant 43 dont la pièce conductrice 44 assure la connexion au moteur. Les pièces 43 et 44 sont usinées en forme de cône 45 dont le sommet est dirigé vers le bas. L'espace entre le boîtier et le cône contient une bille conductrice 42. L'alimentation du moteur est assurée par une batterie 29 en contact avec une pièce conductrice 46 elle-même chassée dans une couronne isolante 47 solidaire du boîtier 12.

Les deux pièces 46 et 47 sont usinées en forme de cône 48 d'un angle approximativement semblable à celui du cône 45 dont le sommet est également dirigé vers le bas.

Si la pression sur la lame 1 est exercée approximativement verticalement au plan de coupe, la bille conductrice 42 se trouvera au centre du cône 45 et mettra en contact les deux parties métalliques 44 et 46 assurant ainsi l'enclenchement du moteur.

Si le boîtier 12 n'est pas tenu perpendiculairement au plan de coupe, la bille 42 quittera la partie centrale conductrice 44. Le moteur ne s'enclenchera pas lors d'une pression sur le boîtier.

Si la lame est dirigée vers le haut, la bille roulera à l'extérieur du cône 48 et l'enclenchement ne sera pas possible non plus.

Une seule position verticale, lame vers le bas, permet la mise en marche du moteur.

Ce dispositif évite donc tout accident lorsque l'opérateur porte la nourriture coupée à la bouche. La sensibilité à la verticalité sera fonction de l'angle α du cône.

Si pour certaines applications, le fonctionnement du contact devait intervenir dans les deux positions verticales du boîtier, il suffirait d'inverser le cône supérieur, c'est-à-dire de le rendre concave plutôt que convexe.

Pour amortir plus rapidement le mouvement de rotation de la bille autour de l'axe du cône 45, on pourra introduire quelques chicanes radiales qui pourraient être réalisées sous forme de rainures radiales fraisées dans la couronne isolante 43.

Une autre forme d'exécution consiste à réduire la largeur de la couronne isolante 43 et à augmenter le diamètre du contact central 44 légèrement saillant. Dans ces conditions, le temps de fermeture du contact est réduit à un minimum.

La fonction recharge doit être, elle aussi, adaptée à l'handicapé; en effet, l'introduction d'une petite fiche dans une prise de courant demande une précision souvent hors de portée de l'handicapé. L'appareil envisagé permet la mise en recharge des batteries, sans motricité fine. Il suffit de poser le couteau, fond en bas, sur un socle d'alimentation, sans se préoccuper d'aucun positionnement ni de liaison.

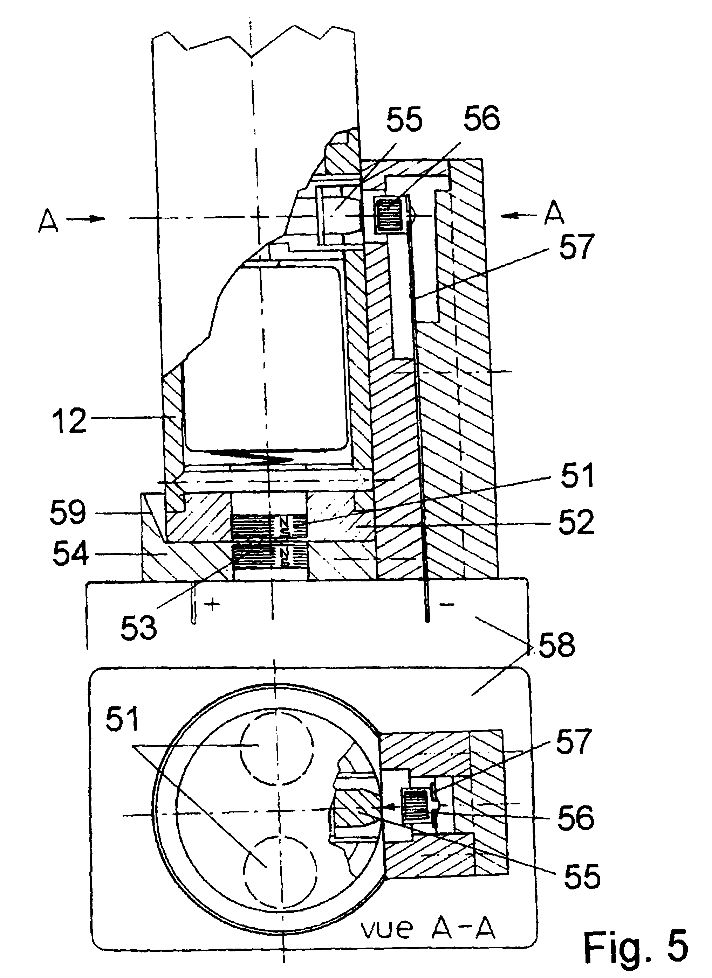

La figure 5 montre un exemple d'exécution de l'appareil de recharge.

Le couteau roratif étant cylindrique, il convient de déterminer la génératrice sur laquelle se trouve la borne de recharge. Ceci est réalisé par une paire d'aimants 51 logés quasiment en surface extérieure du couvercle 52 donnant accès aux accumulateurs d'une part, et une même paire d'aimants 53 également logés quasiment en surface de la partie supérieure d'un support creux 54 conçu pour recevoir le couteau rotatif.

La polarité de chaque paire d'aimants est telle qu'elle impose une position angulaire du couteau mettant la borne de recharge dans la position souhaitée. Si la présentation du couteau rotatif sur son socle n'est pas la bonne, il est repoussé ou va tourner de lui-même dans la position exacte. En effet, les deux aimants 53 sont noyés dans le support 54 sur un même diamètre, l'un ayant son pôle nord dirigé vers le haut, tandis que l'autre son pôle sud dirigé vers le haut.

Dans le couvercle 52 du couteau, les deux autres aimants quasi en surface du fond sont également disposés sur le même diamètre que ceux noyés dans le support. Leur polarité est également inversée et se trouve sur un diamètre perpendiculaire à la génératrice sur laquelle se trouve la borne de recharge. Ainsi, posant le boîtier 12 du couteau sur le support 54, toute erreur d'orientation est aussitôt corrigée. Un couple contraindra la borne d'alimentation 55 en matériau magnétique à se placer face au contact constitué d'un aimant 56, lequel monté sur une lame ressort 57 viendra se coller immédiatement sur la borne de contact 55.

Le support métallique 54 fixé sur un transformateur-redresseur 58 est relié au pôle positif, alors que le contact 56, 57 est relié au pôle négatif, la charge de la batterie intervient sans aucune intervention autre que de placer le couteau sur son socle. Afin de faciliter le placement du dispositif sur son support 54 il peut être prévu un rebord 59 de manière à former une cuvette. La difficile connexion par un Jack pour un handicapé est donc supprimée.

Les formes d'exécution décrites ci-devant ne sont là que pour donner des exemples de réalisation dudit dispositif de table. Elles ne limitent en rien l'invention, notamment en ce qui a trait à la forme du boîtier oblong qui pourrait être autre que circulaire, par exemple hexagonale, carrée, octogonale ou autrement, ainsi qu'à la matière utilisée pour le réaliser qui peut être du métal, de la matière synthétique, des alliages métalliques légers.

## Revendications

1. Dispositif de table pour handicapés physiques, à motorisation et source d'énergie électrique intégrée, destiné à découper une partie d'une pièce de nourriture présentant un boîtier oblong (12) pouvant être tenu d'une seule main, comportant à l'une de ses extrémités un organe de coupe (1) avec des moyens liés à un moteur électrique (2) permettant de mettre en rotation ledit organe, et des moyens (6, 7) pour immobiliser la pièce à découper (8) lors de la rotation dudit organe, caractérisé en ce que ledit organe de coupe (1) est en forme de tube cylindrique circulaire, dont l'extrémité distale présente une partie tranchante (4) et dont l'extrémité proximale (27) est en prise avec des moyens (25, 26) liés audit moteur électrique permettant de le mettre en rotation, et en ce qu'il comporte un élément d'enclenchement (22, 34 ; 42, 43, 44, 45, 46, 47, 48) du moteur agissant par pression sur ledit boîtier oblong (12)

2. Dispositif selon la revendication 1, caractérisé en ce que l'élément d'enclenchement (42, 43, 44, 45, 46, 47, 48) est un contacteur de sécurité ne permettant l'enclenchement du moteur que lorsque le boîtier est dans une position sensiblement verticale avec l'organe de coupe dirigé vers le bas.

3. Dispositif selon la revendication 2, caractérisé en ce que le contacteur de sécurité est constitué par une bille (42) de métal conducteur logée dans un logement conique concave (45), dont la partie centrale (44) proche du sommet est conductrice et dont le pourtour (43) est isolant, le logement conique comportant un couvercle en forme de cône convexe (48), dont la partie centrale (46) proche du sommet est conductrice et dont le pourtour (47) est isolant, qui peut être mis en appui sur la bille à l'encontre d'une force donnée par un premier ressort (9) de manière à fermer le circuit d'alimentation du moteur d'entraînement de l'organe de coupe (1).

4. Dispositif selon l'une des revendications 1 à 3, caractérisé en ce que l'organe de coupe (1) comporte à sa partie proximale (27) un aimant (25) assurant son maintien dans le boîtier oblong (12) et son entraînement en rotation de manière à pouvoir être retiré notamment pour son nettoyage, ledit aimant maintenant aussi à l'intérieur de l'organe de coupe un second ressort (24) à l'extrémité duquel est disposé un piston (23) sensiblement au niveau de l'extrémité distale dudit organe de coupe (1) et coulissant dans ce dernier de manière à constituer un extracteur des parties découpées.

5. Dispositif selon l'une des revendications précédentes, caractérisé en ce que les moyens pour immobiliser la pièce à découper sont constitués par un cylindre (5) extérieur et concentrique à l'organe de coupe (1) comportant des dents (6) ou des pics (7) à sa partie distale et des dents (15) à sa partie proximale de manière à le fixer en rotation relativement avec un boîtier cylindrique (19) comportant le moteur lui-même fixé en rotation avec le boîtier oblong (12).

6. Dispositif selon la revendication 5, caractérisé en ce que le cylindre (5) concentrique à l'organe de coupe est maintenu en place par des aimants (14) disposés sur une bague (13) porte-couronne liée en rotation avec le boîtier cylindrique (19) comportant le moteur et mobile en déplacement axial relativement au boîtier oblong (12) et maintenu dans une position déterminée par un ressort (9).

7. Dispositif selon l'une des revendications 1 à 4, caractérisé en ce que les moyens pour immobiliser la pièce à découper sont constitués par une fourchette (38) disposée à l'intérieur de l'organe de coupe (1, fig. 3) et maintenue fixe en rotation avec le boîtier oblong (12).

8. Dispositif selon l'une des revendications 1 à 4, caractérisé en ce que l'ensemble (19a, fig. 2a) constitué par le moteur et l'organe de coupe est monté sur deux articulations tenues par deux bras (2a, 3a) eux-mêmes articulés de manière indépendante par rapport à un manche (12a).

9. Dispositif selon l'une des revendications 1 à 4, caractérisé en ce que l'organe de coupe (1b, fig. 2b) est entraîné par une rotule (2b) solidaire en rotation avec le moteur (19b).

10. Dispositif selon l'une des revendications 1 à 4, caractérisé en ce que l'organe de coupe (1 c, fig. 2c) comporte à son extrémité distale deux articulations (15c, 16c) décalées de 90°.

11. Dispositif selon l'une des revendications 1 à 4, caractérisé en ce que l'organe de coupe (1d, fig. 2d) comporte une partie (18d) saillante et rétractable élastiquement.

12. Dispositif selon l'une des revendications précédentes, caractérisé en ce qu'il comporte un groupe moto-réducteur constitué du moteur (2), et d'un réducteur (3).

13. Dispositif selon l'une des revendications précédentes, caractérisé en ce que le boîtier oblong (12) comporte à l'extrémité opposée à l'organe de coupe deux pastilles aimantées (51 )de polarités différentes et des moyens de connexion (55) pour la recharge de la batterie destinés à venir se placer sur un appareil de recharge.

14. Appareil de recharge pour un dispositif de table selon la revendication 13, caractérisé en ce qu'il comprend un support creux (54, 58) conçu pour recevoir l'extrémité du boîtier oblong (12) opposée à l'organe de coupe du dispositif, ledit support présentant deux pastilles aimantées (53) de polarités différentes de manière à ce que quel que soit la position du dispositif sur le support, ledit dispositif étant ramené et maintenu dans une position déterminée pour que ses moyens de connexion entre en contact avec des bornes correspondantes prévues sur l'appareil.

## Patentansprüche

1. Tischvorrichtung für körperbehinderte Menschen, mit integrierter Motorisierung und integrierter elektrischer Energiequelle zum Zerschneiden von Nahrungsmittelstücken, welche aus einem länglichen Gehäuse besteht, welches einhändig gehalten werden kann, dadurch gekennzeichnet, dass das Gehäuse an einem Ende ein Schneidorgan in der Form eines zylindrischen, runden Rohrs umfasst, dessen entferntes Ende eine Schneidvorrichtung aufweist, und dessen anderes Ende mit den Vorrichtungen eines elektrischen Motors in Eingriff ist, der das Organ zu drehen vermag, sowie aus Mitteln zum Festhalten des zu zerschneidenden Nahrungsmittels während des Drehens des besagten Organs, und einem Element zum Einschalten des Motors durch Druck auf das besagte Gehäuse.

2. Vorrichtung nach Anspruch 1, dadurch gekennzeichnet, dass das Element zum Einschalten ein Sicherheitsschalter ist, der es nur dann ermöglicht, den Motor einzuschalten, wenn sich das Gehäuse in einer nahezu vertikalen Position, mit dem Schneidwerkzeug nach unten zeigend, befindet.

3. Vorrichtung nach Anspruch 2, dadurch gekennzeichnet, dass der Sicherheitsschalter aus einer stromleitenden Metallkugel besteht, die in einem konkav gestalteten, konischen Gehäuse untergebracht ist, wobei der zentrale, obere Teil stromleitend und der Umfang ansonsten nichtleitend ist, wobei das konische Gehäuse einen konischen, konvexen Deckel aufweist, dessen zentraler, oberer Teil nahe dem höchsten Punkt stromleitend ist, und dessen Umfangsbereich nichtleitend ist, und der mit Hilfe einer, über eine erste Feder vermittelte Kraft gegen die Kugel gedrückt werden kann, um den Versorgungskreislauf des Antriebsmotors für die Schneidwerkzeuge zu schließen.

4. Vorrichtung nach einem der Ansprüche 1 bis 3, dadurch gekennzeichnet, dass das Schneidorgan an seinem dem Gehäuse zugewandten Ende einen Magnet aufweist, welcher die Halterung des Schneidorgans im Gehäuse und dessen Antrieb für die Drehbewegung sichert, um insbesondere zu Reinigungszwecken abgenommen werden zu können, und wobei der Magnet ebenfalls im Innern des Schneidorgans eine zweite Feder hält, an deren Ende ein Zylinder angeordnet ist, der sich nahezu auf gleicher Höhe wie das ferne Ende des besagten Organs befindet und der sich im Schneidorgan hin- und herbewegt, und so die geschnittenen Stücke extrahiert.

5. Vorrichtung nach einem der vorstehenden Ansprüche, dadurch gekennzeichnet, dass die Mittel zum Verriegeln des zu schneidenden Teils aus einem Zylinder, ausserhalb des Schneidorgans und mit letzterem konzentrisch angeordnet, bestehen, der an seinem äusseren Ende mit Zähnen oder Spitzen bestückt ist, und an seinem anderen Ende Zähne besitzt, um diesen durch Drehung an einem zylindrischen Gehäuse zu führen, welches den Motor beinhaltet, der selbst rotationsgebunden auf dem länglichen Gehäuse befestigt ist.

6. Vorrichtung nach Anspruch 5, dadurch gekennzeichnet, dass der mit dem Schneidorgan konzentrische Zylinder durch Magnete gehalten wird, die auf einer Steckkopf-Aufnahme angeordnet sind, rotationsgebunden mit dem zylindrischen Gehäuse, welches den Motor beinhaltet, und längs des länglichen Gehäuses beweglich ist und durch eine dritte Feder in einer bestimmten Position festgehalten wird.

7. Vorrichtung nach einem der Ansprüche 1 bis 4, dadurch gekennzeichnet, dass die Mittel für das Verriegeln des zu schneidenden Teils aus einer Gabel (10, Abb. 3) bestehen, die im Innern der Schneidvorrichtung (1, Abb. 3) angeordnet ist und die auf dem länglichen Gehäuse rotationsgebunden befestigt ist.

8. Vorrichtung nach einem der Ansprüche 1 bis 4, dadurch gekennzeichnet, dass die Einheit (19a), die durch den Motor und das Schneidorgan gebildet wird, auf zwei Gelenke montiert ist, die durch zwei Arme (2a, 3a) gehalten werden, die auf unabhängige Weise über eine Haltestange (6a) selbst gelenkig verbunden sind.

9. Vorrichtung nach einem der Ansprüche 1 bis 4, dadurch gekennzeichnet, dass das Schneidorgan (1b) durch ein rotationsgebunden mit dem Motor (19b) einstückiges Gelenk (2b) angetrieben wird.

10. Vorrichtung nach einem der Ansprüche 1 bis 4, dadurch gekennzeichnet, dass das Schneidorgan (1c) an seinem äusseren Ende zwei um 90° versetzte Gelenke (15c, 16c) aufweist.

11. Vorrichtung nach einem der Ansprüche 1 bis 4, dadurch gekennzeichnet, dass das Schneidorgan (1d) einen vorstehenden und elastisch zurückziehbaren Teil (18d) aufweist.

12. Vorrichtung nach einem der vorstehenden Ansprüche, dadurch gekennzeichnet, dass sie eine Getriebemotorengruppe mit einem Motor und einem Getriebe (Untersetzungsgetriebe) aufweist.

13. Vorrichtung nach einem der vorstehenden Ansprüche, dadurch gekennzeichnet, dass das längliche Gehäuse am dem Schneidorgan gegenüberliegenden Ende zwei magnetische Plättchen unterschiedlicher Polaritäten und Anschlussmittel zum Aufladen der Batterie aufweist, die für die Anordnung in einem Ladegerät bestimmt sind.

14. Ladegerät für eine Tischvorrichtung nach Anspruch 13, dadurch gekennzeichnet, dass es eine hohle Aufnahme enthält, die so gestaltet ist, um die, dem Schneidorgan gegenüberliegende Seite der Vorrichtung aufzunehmen, wobei die benannte Aufnahme zwei magnetische Plättchen unterschiedlicher Polaritäten besitzt, um zu erreichen, dass, gleich welche Position die Vorrichtung in der Aufnahme einnimmt, diese Vorrichtung in eine bestimmte Position gebracht und auch dort gehalten wird, um zu ermöglichen, dass die Anschlussmittel mit den entsprechenden, auf dem Apparat vorgesehenen Klemmen in Kontakt treten.

## Claims

1. A meal-table implement for the physically handicapped, with integrated motor drive and power source, for cutting off a piece of food, comprising an oblong casing which can be held in one hand,
characterized in that
it comprises at one of its ends a cutting device in the form of a cylindrical tube of which the distal circular end is fitted with a cutting part and of which the proximal end engages means linked to an electric motor allowing to drive said cutting device, and a motor-engaging means actuated by pressing on said case.

2. Apparatus as claimed in claim 1, characterized in that the motor-engaging means is a safety contactor allowing motor-engagement only when the casing is in a substantially vertical position and the cutting device is pointing downward.

3. Apparatus as claimed in claim 2, characterized in that the safety contactor consists of a conducting metal-ball housed in a concave conical housing of which the middle part near the apex is conducting and of which the periphery is insulating, the conical housing comprising a cover in the form of a convex cone of which the middle part near the apex is conducting and of which the periphery is insulating and which can be made to contact the ball to oppose a force exerted by a first spring in such manner as to close the power-supply circuit of the drive motor of the cutting device.

4. Apparatus as claimed in any of claims 1 through 3, characterized in that at its proximal zone the cutting device comprises a magnet to hold it in place in the case and to drive it in rotation in such manner that it can be withdrawn, in particular for purposes of cleaning, said magnet also holding in place, inside the cutting device, a second spring at the end of which is mounted a piston located substantially at the distal end of said cutting device and sliding in the cutting device so as to constitute an extraction means for the cutoff parts.

5. Apparatus as claimed in any of the above claims, characterized in that the means to hold in place the food from which to cut off a piece consist of an outer cylinder concentric with the cutting device fitted at its distal end with teeth or tines and with teeth at its proximal portion in such manner as to irrotationally affix said cylinder on a cylindrical case comprising the motor which in turn is irrotationally mounted to the oblong cylinder.

6. Apparatus as claimed in claim 5, characterized in that the cylinder concentric with the cutting device is held in place by magnets mounted on a collar-supporting ring irrotationally connected to the cylindrical case holding the motor and axially displaceable relative to the oblong case and kept in a specific position by a third spring.

7. Apparatus as claimed in one of claims 1 through 4, characterized in that the means immobilizing the food to be cut consist of a fork (10, Fig. 3) mounted inside the cutting device (1, Fig. 3) and kept irrotational relative to the oblong casing.

8. Apparatus as claimed in one of claims 1 through 4, characterized in that the sub-assembly (19a) composed of the motor and the cutting device is mounted on two articulations which are held by two arms (2a, 3a) themselves independently hinging on a sleeve (6a).

9. Apparatus as claimed in any of claims 1 through 4, characterized in that the cutting device (1b) is driven by a universal joint (2b) irrotationally affixed to the motor (19b).

10. Apparatus as claimed in one of claims 1 through 4, characterized in that the cutting device (1c) comprises at its distal end two joints (15c, 16c) offset by 90°.

11. Apparatus as claimed in one of claims 1 through 4, characterized in that the cutting device (1d) comprises an elastically retractable and projecting portion (18d).

12. Apparatus as claimed in one of the above claims, characterized in that it comprises a gear-motor unit consisting of the motor and a gear reducer.

13. Apparatus as claimed in one of the above claims, characterized in that at its end opposite the cutting device the oblong case comprises two magnetized pellets of different polarities and connectors to recharge the battery, said connectors being designed to fit on a recharging device.

14. Recharging device for a meal-table implement as claimed in claim 13, characterized in that it comprises a hollow support designed to receive that end which is opposite the apparatus' cutting device, said support having two magnetized pellets of different polarities in such manner that regardless of the apparatus' position on the support, said apparatus shall be brought back to and kept in a specific position so that its connectors shall make contact with corresponding terminals fitted on the apparatus.
